# EUROPEAN PATENT SPECIFICATION

(11) **EP 2 937 422 B1**
(45) Date of publication and mention of the grant of the patent: **18.12.2019**
(21) Application number: 15164907.6
(22) Date of filing: 23.04.2015
(51) Int. Cl.: C12Q 1/6827, C12Q 1/6883

(54) **A METHOD AND A KIT FOR NON-INVASIVELY DETECTING FETAL DEAFNESS PATHOGENIC GENE MUTATIONS**
VERFAHREN UND KIT ZUR NICHTINVASIVEN DETEKTION VON PATHOGENEN GENMUTATIONEN DER FÖTUSTAUBHEIT
PROCÉDÉ ET KIT PERMETTANT DE DÉTECTER DE MANIÈRE NON INVASIVE DES MUTATIONS DE GÈNES PATHOGÈNES DE SURDITÉ F TAL

(30) Priority: 23.04.2014 CN 201410174277
(43) Date of publication of application: 28.10.2015
(73) Proprietor: Berry Genomics Co., Ltd, Chaoyang District Beijing 100015 (CN)
(72) Inventor: Liu, Yiqian, 100015 Beijing (CN); Guo, Xianchao, 100015 Beijing (CN); Fu, Yong, 100015 Beijing (CN); Hu, Yugang, 100015 Beijing (CN); Li, Tiancheng, 100015 Beijing (CN); Zhang, Jianguang, 100015 Beijing (CN)
(74) Representative: Plasseraud IP

(56) References cited:
- WO-A1-2014/008635
- WO-A2-2012/162267
- CN-A- 103 276 065
- CN-A- 103 436 609
- CN-B- 102 534 031
- FENG XIN ET AL: "Genetic mutations in nonsyndromic deafness patients of Chinese minority and han ethnicities in Yunnan, China", JOURNAL OF TRANSLATIONAL MEDICINE, BIOMED CENTRAL, LONDON, GB, vol. 11, no. 1, 17 December 2013 (2013-12-17), page 312, XP021171384, ISSN: 1479-5876, DOI: 10.1186/1479-5876-11-312

## Description

### Field of Invention

The present invention relates to genetic diagnosis field. More specifically, the present invention is directed to a method for detecting fetal deafness pathogenic gene mutations. The present invention also relates to a kit and uses thereof for detecting fetal deafness pathogenic gene mutations.

### Background of Invention

Deafness is a common disease resulting in disability and affecting health in humans, and is also one of the most common genetic diseases in clinic. According to statistics, there is one with dysaudia among every 1000 new-born babies over the world (Steel K P. New interventions in hearing impairment [J]. BMJ ,2000 ,320 (4) :622∼625). Many reasons can result in deafness, while the genetic factor is the main one. Hearing disability occurred at the time of born or before 3 years old is called pre-lingual hearing impairment, at least half of which is caused by genetic deficiency. In a large number of patients with delayed hearing loss, many patients developed said disease due to their genetic deficiency, or due to increased susceptibility to the environment caused by genetic deficiency and polymorphism. It is estimated that there are totally more than 100 non-syndrome genetic deafness genes globally. In view of the domestic research progress, most of the pathogenicity loci found in China are on GJB2, GJB3, SLC26A4 and mitochondria 12SrRNA.

Brief introductions of GJB2, GJB3, and SLC26A4 are provided herein for better understanding. GJB2 gene: this gene is located on autosomal 13q11-12 region, and the DNA full length is 4804 bp, including 2 exons. The coding region is 678 bp, and encodes a connexion Connexin26 consisting of 266 amino acid residues, which belongs to beta-2 protein that is part of the potassium circulation pathway. GJB2 gene mutation is the most common cause for genetic deafness, and the deafness resulted from GJB2 gene mutation is pre-lingual, bilateral, and symmetric deafness, which varies a lot in terms of the degree of hearing loss. It may range from mild to extremely severe, while most is severe or extremely severe deafness. In Chinese population, the main types of GJB2 gene mutation are 235delC, 299-300delAT, 176-191de116 and the like, accounting for over 80% of the populations with GJB2 gene mutation. GJB3 gene: this gene is located on autosomal 1q33-35 region, has 2 exons, and encodes a connexion Connexin 31 comprising 270 amino acids. GJB3 gene mutation can lead to autosomal genetic non-syndrome deafness, dominant or recessive, and is considered to be associated with high frequency hearing loss. SLC26A1 gene is located on autosomal 7q31 region, has 21 exons, and encodes a multipass transmembrane protein Pendrin consisting of 780 amino acid residues, which belongs to the transporter family that mainly relates with iodine/chloride ion transportation and plays an important role in maintaining the balance of the body ion compositions. Recently, many studies abroad demonstrate that SLC26A4 is close related with Pendred syndrome (enlarged vestibular aqueduct or accompanied with nerve deafness of inner ear malformation and goiter) and large vestibular aqueduct syndrome (LVAS). Among the numerous mutations, many are presented in both Pendred syndrome and LVAS. Thus, mutations at the same loci may result in different clinic performances. There are many types of SLC26A4 gene mutations, while the mutation frequencies of 281C>T, 589G>A, IVS7-2A>G, 1174A>T(N392Y), 1226G>A, 1229C>T(T410M), 1975G>C, 2027T>A(L676Q), 2168A>G(HIS723ARG) and IVS15+5G>A are as high as 82.51%.

With the development of science and technology, many deafness patients of new-born babies are diagnosed using methods of Sanger sequencing, gene chip and protein detection. There is also prenatal detection based on invasive diagnosis of fetal deafness pathogenic genes. However, non-invasively detection of fetal deafness pathogenic genes has not been achieved yet.

### Summary of Invention

The inventor explored a method for detecting fetal deafness pathogenic gene mutations (genotype) using fragment DNA from venous blood of a pregnant woman based on the second generation high-throughput sequencing technology. After the discovery of embryo DNA in maternal blood, it is possible to non-invasively diagnose and detecting fetal chromosomal abnormalities and gene mutations directly (Lo YM et al., (1997) Presence of fetal DNA in maternal plasma and serum. Lancet, 350:485-487). The Illumina products are the best among the second generation high-throughput sequencing technologies, in which two representative products are Miseq and Hiseq, one is known for sequencing length, and the other is known for sequencing throughput. Miseq sequencer is used in the present invention. However, the amount of embryo DNA in blood is low, and how to determine the related fetal genotypes quickly and accurately is still a technical problem to be solved.

The technical problem to be solved in the present invention is how to non-invasively detecting the genotypes of fetal deafness pathogenic genes using the venous blood of a pregnant woman. Accordingly, the first object of the present invention as defined by the appended claims is to provide a method that is capable of effectively detecting gene mutations of pre-determined mutation loci on deafness pathogenic genes in a fetus through the use of plasma DNA samples, comprising the following steps:
a) designing primers according to a plurality of pre-determined mutation loci of deafness pathogenic genes comprising 22 loci of GJB2 gene, GJB3 gene and SLC26A4 gene;
b) simultaneously testing the plurality of pre-determined mutation loci of deafness pathogenic genes by:
   i) connecting plasma DNAs contained in a plasma DNA sample obtained from a pregnant woman with pre-amplification barecode linkers to obtain connected products by labeling the start and terminal positions of the plasma DNAs and 6 different bases on 3' end of the pre-amplification linker;
   ii) PCR-pre-amplifying the connected product to obtain pre-amplified products;
   iii) cyclizing the pre-amplified product to obtain cyclized DNAs;
   iv) PCR amplifying the cyclized DNAs using the designed primers to obtain amplified products; and
   v) high throughput sequencing the amplified products and detecting mutations of the fetal deafness pathogenic genes, wherein the primers are at least one pair of primers that are backward extended and are:
      GJB2-F1 (SEQ ID NO: 2): CACGCTGCAGACGATCC
      GJB2-R1 (SEQ ID NO: 3): CCCCAATCCATCTTCTACTCT
      GJB2-F2 (SEQ ID NO: 4): TCCCACATCCGGCTATG
      GJB2-R2 (SEQ ID NO: 5): GATGGGGAAGTAGTGATCGTAG
      GJB3-F (SEQ ID NO: 7): CGTGGACTGCTACATTGCC
      GJB3-R (SEQ ID NO: 8): ATGTTGGGGCAGGGG
      PDS3-F (SEQ ID NO: 10): CGTCATTTCGGGAGT
      PDS3-R (SEQ ID NO: 11): CTAAGCAGCCATTCC
      PDS5-F (SEQ ID NO: 13): CCCTGACTCTGCTGG
      PDS5-R (SEQ ID NO: 14): CACTGGCAATCAGGA
      PDS7-F2 (SEQ ID NO: 16): TGGCAGTAGCAATTATCGTC
      PDS7-R2 (SEQ ID NO: 17): TTTCATATGGAGCCAACCTG
      PDS10-F (SEQ ID NO : 19): CCACTGCTCTTTCCCGC
      PDS10-R (SEQ ID NO: 20): CAAGAGAAGAATCCTGAGAAGATG
      PDS17-F (SEQ ID NO : 22): TTCCTGGACGTTGTTGGAG
      PDS17-R (SEQ ID NO : 23): GATATAGCTCCACAGTCAAGCAC
      PDS19-F (SEQ ID NO : 25): TCTTGAGATTTCACTTGGTT
      PDS19-R (SEQ ID NO : 26): GTTCCATTTTAGAAACGGTA.

According to one preferred embodiment of the invention, detection of the 22 loci of GJB2 gene, GJB3 gene and SLC26A4 gene is accomplished in one PCR using 9 pairs of primers.

According to one preferred embodiment of the invention, there are at least two base differences between the linkers.

According to one preferred embodiment of the invention, the linkers are partially matched Y-type linkers.

According to one preferred embodiment of the invention, the method used in the cyclization of step c) is a splint cyclization, wherein single stand DNAs complementary to both ends of the pre-amplified DNA are used as splints; and close of the ring is completed by a heat-resistant Taq ligase.

According to one preferred embodiment of the invention, the cyclization of step c) is multiple reactions of a single system circulation consisting of DNA denaturation, splint DNA annealing and connecting.

According to one preferred embodiment of the invention, the method of the invention further comprises digesting the uncyclized linear DNA.

According to one preferred embodiment of the invention, the method of the invention detects the genotype of fetal deafness pathogenic genes using fragment DNA from venous blood of a pregnant woman.

According to one preferred embodiment of the invention, the deafness pathogenic genes have insertion, deletion, substitution or gene fusion mutations.

The second object of the present invention as defined by the appended claims is to provide a kit for non-invasively detecting fetal deafness pathogenic gene mutations, comprising: reagents for extracting plasma DNAs, a barecode linker for labeling the extracted plasma DNAs through the start and terminal positions of the extracted plasma DNAs and 6 different bases on 3' end of the linker, a DNA cyclase, primers and reagents for amplifying target DNAs, and primers and reagents for pre-amplifying the pre-determined loci of deafness pathogenic genes, wherein the primers for pre-amplifying the pre-determined loci of deafness pathogenic genes are a pair of primers that are backward extended and designed to simultaneously testing a plurality of mutation loci comprising 22 loci of GJB2 gene, GJB3 gene and SLC26A4 gene, the pair of primers being:
GJB2-F1 (SEQ ID NO: 2): CACGCTGCAGACGATCC
GJB2-R1 (SEQ ID NO: 3): CCCCAATCCATCTTCTACTCT
GJB2-F2 (SEQ ID NO: 4): TCCCACATCCGGCTATG
GJB2-R2 (SEQ ID NO: 5): GATGGGGAAGTAGTGATCGTAG
GJB3-F (SEQ ID NO: 7): CGTGGACTGCTACATTGCC
GJB3-R (SEQ ID NO: 8): ATGTTGGGGCAGGGG
PDS3-F (SEQ ID NO: 10): CGTCATTTCGGGAGT
PDS3-R (SEQ ID NO: 11): CTAAGCAGCCATTCC
PDS5-F (SEQ ID NO: 13): CCCTGACTCTGCTGG
PDS5-R (SEQ ID NO: 14): CACTGGCAATCAGGA
PDS7-F2 (SEQ ID NO: 16): TGGCAGTAGCAATTATCGTC
PDS7-R2 (SEQ ID NO: 17): TTTCATATGGAGCCAACCTG
PDS10-F (SEQ ID NO: 19): CCACTGCTCTTTCCCGC
PDS10-R (SEQ ID NO: 20): CAAGAGAAGAATCCTGAGAAGATG
PDS17-F (SEQ ID NO: 22): TTCCTGGACGTTGTTGGAG
PDS17-R (SEQIDNO: 23): GATATAGCTCCACAGTCAAGCAC
PDS19-F (SEQ ID NO: 25): TCTTGAGATTTCACTTGGTT
PDS19-R (SEQ ID NO: 26): GTTCCATTTTAGAAACGGTA.

### Brief Description of Figures

Fig. 1 shows the design of primers used in the present invention.
Fig. 2 shows the structure of connected products of the present invention.
Fig. 3 shows auxiliary sequence and linker sequence of the present invention, wherein the arrow points to the position needed to be linked by Taq DNA ligase.

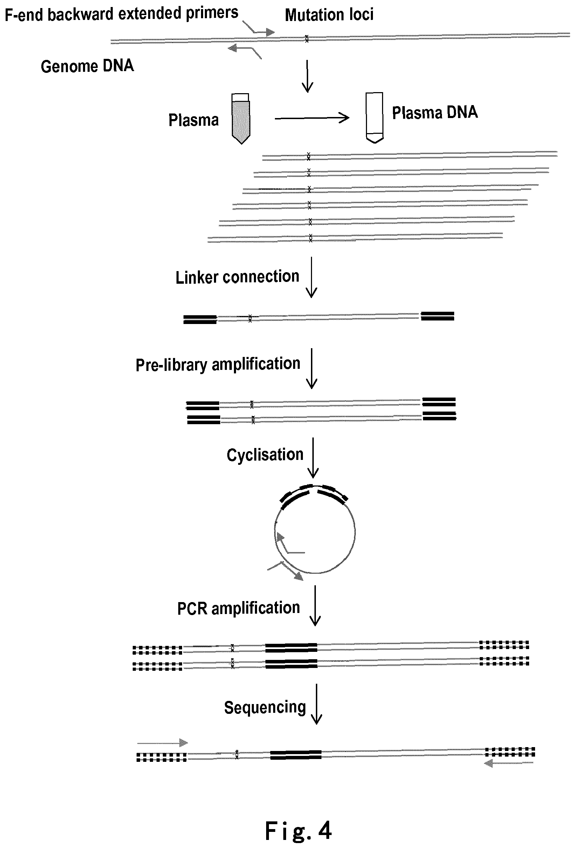

Fig. 4 shows one preferred embodiment of the method of the invention, illustrating the scheme of constructing a library.

### Detailed Description of the Invention

The first object of the present invention is to provide a method for detecting gene mutations of pre-determined mutation loci of deafness pathogenic genes in a fetus through the use of plasma DNA samples, comprising the steps as defined herein above.

Traditional methods for detecting fragment DNA are mainly performed by PCR amplifying the regions to be tested before detection. As the PCR primers are on both ends of the regions to be tested, the regions to be tested are required to be complete. However, the regions to be tested in most fragment DNAs are incomplete, as the fragment DNAs are produced by random cleavage. Accordingly, the number of fragment DNAs that can be used as amplification templates is few, and is difficult to be detected. In the invention, the adaptation range of the primer amplification and the effective amount of templates are largely increased by cyclizing the fragment DNAs, thereby improving the detection sensitivity of the fragment DNA dramatically. A more important aspect of the invention is to label the initial template using the start and terminal positions of the fragment DNA as well as the 6 different bases on 3' end of the linker while improving the utility of the template. Thus, whether the pre-determined loci in mother and fetus have been mutated as well as the type of mutations can be effectively determined. Another important aspect of the invention is to detect multiple loci to be tested simultaneously, e.g., to detect the 22 high frequency pathogenic loci in a sample to be tested at one time using the Multi-PCR technology.

The detection method of the invention can be used to detect fragment DNA comprising mutations, such as homozygous mutation and heterozygous mutation; or base deletion, insertion or substitution. "Fragment DNA" of the invention refers to short fragment DNA with the length of about 166bp that is formed by random cleavage of the genome DNA of an organism.

In sum, template cyclization of the invention makes the available DNA templates more than that of conventional PCR, thereby improving detection sensitivity. Labelling initial templates using the start and terminal positions of the fragment DNA as well as the 6 different bases of Barcode on 3' end of the linker makes it possible to effectively reduce the sequence to a template sequence through sequencing before PCR amplification, thus decreasing the sequence bias resulted from PCR amplification, and determining whether the pre-determined loci in mother and fetus have been mutated as well as the type of mutations more accurately. Meanwhile, 9 pairs of backward extended primers are designed and optimized against high frequency mutation loci of the deafness pathogenic genes GJB2, GJB3, and SLC26A4, and the 22 high frequency pathogenic loci in a sample to be tested are detected at one time using the Multi-PCR technology.

### Experiment design of a deafness example

### 1. Primer design of the pre-determined mutation loci on deafness pathogenic genes.

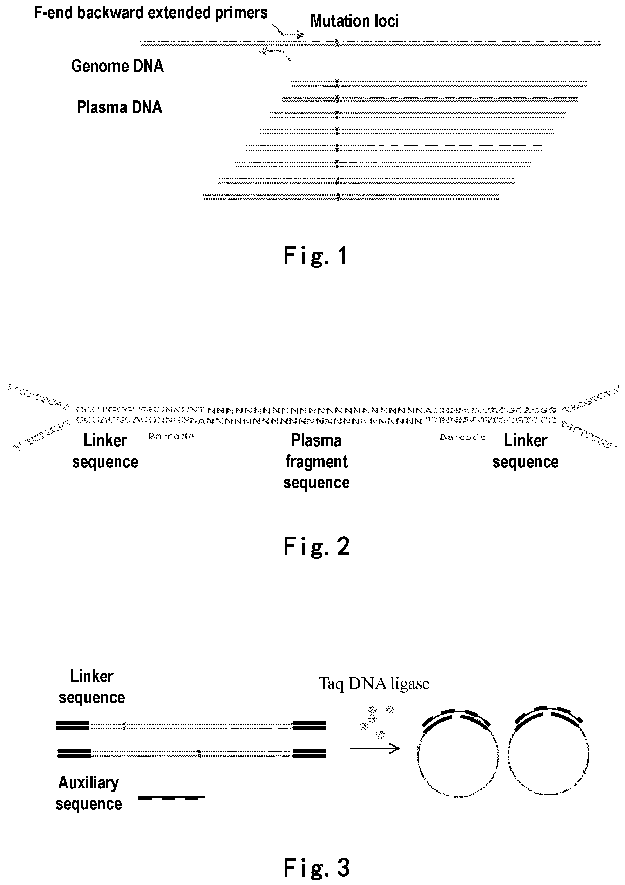

To achieve the detection of mutation genotypes of the pre-determined loci of fetal deafness pathogenic genes in maternal venous blood, the mutation of the pre-determined loci of the invention is at least one gene mutation selected from the 22 loci of GJB2 gene, GJB3 gene, and SLC26A4 gene, preferably, the mutation of GJB2 gene is at least one mutation selected from 35delG, 109G>A(VAL37ILE)167delT, 176-191dell6, 235delC and 299_300delAT,; the mutation of GJB3 gene is at least one mutation selected from 421A>G(ILE141VAL), 421-423delATT, 497A-G(ASN166SER), 538C>T(ARG180TER), 547G>A(GLU183LYS) and 580G>A(ALA194THR); and the mutation of SCL26A4 gene is at least one mutation selected from 281C>T, 589G>A, IVS7-2A>G, 1174A>T(N392Y), 1226G>A, 1229C>T(T410M), 1975G>C, 2027T>A(L676Q), 2162C>T(T721M), 2168A>G(HIS723ARG) and IVS15+5G>A. The tested loci do not include mitochondria C1494T and A1555G mutation loci, as the amount of mitochondria DNA in plasma is very low. More preferably, the pairs of primers are designed to extend backward for detection of pre-determined mutation loci, and the F-end primer is closer to the tested loci, as shown in Fig. 1. More preferably, the 22 loci on GJB2, GJB3, and SLC26A4 genes are detected in one PCR using 9 pairs of primers by the mean of Multi-PCR that solves the multi-point detection. The specific sequences tested in GJB2, GJB3, and SLC26A4 genes and the primers are as follows:
Backward extended amplification primers in the target region aimed at exon 2 of GJB2.
The sequence of GJB2 exon 2 is as follows (SEQ ID NO: 1):
GJB2-F1 (SEQ ID NO: 2): CACGCTGCAGACGATCC
GJB2-R1 (SEQ ID NO: 3): CCCCAATCCATCTTCTACTCT
GJB2-F2 (SEQ ID NO: 4): TCCCACATCCGGCTATG
GJB2-R2 (SEQ ID NO: 5): GATGGGGAAGTAGTGATCGTAG

Backward extended amplification primers in the target region aimed at exon 2 of GJB3.
The sequence of GJB3 exon 2 is as follows (SEQ ID NO: 6):
GJB3-F (SEQ ID NO: 7): CGTGGACTGCTACATTGCC
GJB3-R (SEQ ID NO: 8): ATGTTGGGGCAGGGG

Backward extended amplification primers in the target region aimed at exon 3 of SLC26A4.
The sequence of SLC26A4 exon 3 is as follows (SEQ ID NO: 9):
PDS3-F (SEQ ID NO: 10): CGTCATTTCGGGAGT
PDS3-R (SEQ ID NO: 11): CTAAGCAGCCATTCC

Backward extended amplification primers in the target region aimed at exon 5 of SLC26A4.
The sequence of SLC26A4 exon 5 is as follows (SEQ ID NO: 12):
PDS5-F (SEQ ID NO: 13): CCCTGACTCTGCTGG
PDS5-R (SEQ ID NO: 14): CACTGGCAATCAGGA

Backward extended amplification primers in the target region aimed at exon 7, intron 7 and exon 8 of SLC26A4.
The sequence of SLC26A4 exon 7, intron 7 and exon 8 are as follows (SEQ ID NO: 15):
PDS7-F2 (SEQ ID NO: 16): TGGCAGTAGCAATTATCGTC
PDS7-R2 (SEQIDNO: 17): TTTCATATGGAGCCAACCTG

Backward extended amplification primers in the target region aimed at exon 10 of SLC26A4.
The sequence of SLC26A4 exon 10 is as follows (SEQ ID NO: 18):
PDS10-F (SEQ ID NO: 19): CCACTGCTCTTTCCCGC
PDS10-R (SEQ ID NO: 20): CAAGAGAAGAATCCTGAGAAGATG

Backward extended amplification primers in the target region aimed at exon 17 of SLC26A4.
The sequence of SLC26A4 exon 17 is as follows (SEQ ID NO: 21):
PDS17-F (SEQ ID NO: 22): TTCCTGGACGTTGTTGGAG
PDS17-R (SEQ ID NO: 23): GATATAGCTCCACAGTCAAGCAC

Backward extended amplification primers in the target region aimed at exon 19 of SLC26A4.
The sequence of SLC26A4 exon 19 is as follows (SEQ ID NO: 24):
PDS19-F (SEQ ID NO: 25): TCTTGAGATTTCACTTGGTT
PDS19-R (SEQ ID NO: 26): GTTCCATTTTAGAAACGGTA

### 2. Linker design of the plasma DNA

To achieve quantified template detection of the loci of the deafness pathogenic genes, the invention employed barcode (multiple sequence labelled) linkers to label the sequences of plasma DNA. The labelling is obtained using the start and terminal positions of the fragment DNA as well as the 6 different bases on 3' end of the linker. The above labelling achieves two objectives: one is to quantitatively label the initial templates of the tested loci; the other is to exclude contamination between different samples in the same batch. To achieve the first objective, the number of combination types of a group of barcode linkers has to be much bigger than the maximal number of the templates with the same start and same terminal positions. For instance, in 1ml plasma, the maximal number of the templates with the same start and same terminal positions is approximately 10, and the number of combination types of each group of barcode linkers of the invention is 10, thus the number of random combinations of upstream and downstream of the linkers is 100. The combination number of 100 is much bigger than the template number of 10, thus each template is ensured to link to different type of barcode linkers. To achieve the second objectives, various combinations of barcode linkers are designed in the invention. There are totally 16 groups of barcode linkers without repetition between each group of linkers, thus a maximal of 16 samples can be detected in one experiment. More preferably, there are at least two base differences between different barcode linkers, thus to reduce the possibility of mistake. Correction function of the barcode linkers achieves quantification and eliminates contamination. Thus, whether the pre-determined loci in mother and fetus have been mutated as well as the type of mutations can be effectively determined. The specific linker and primer sequences are as follows:
Linker design, need to be annealed to a double strand:
   ssCycAB-1(SEQ ID NO: 27): GTCTCATCCCTGCGTG(NNNNNNT)
   ssCycAB-2(SEQ ID NO: 28): p(NNNNNN)CACGCAGGGTACGTGT
   wherein N can be any amino acid.
pre-library amplification primers :
   ssCycUniprimer-F(SEQ ID NO: 29): GTCTCATCCCTGCGTG
   ssCycUniprimer-R(SEQ ID NO: 30): ACACGTACCCTGCGTG

### 3. Cyclisation of the pre-library

Traditional methods for detecting fragment DNA are mainly performed by PCR amplifying the regions to be tested before detection. As the PCR primers are designed on both ends of the regions to be tested, the regions to be tested are required to be complete. However, the regions to be tested in most fragment DNAs are incomplete, as the fragment DNAs are produced by random cleavage. Accordingly, the number of fragment DNAs that can be used as amplification templates is few, and is difficult to be detected by PCR. The invention uses plasma DNA fragment (about 166bp) to achieve quantified template detection of the loci on the deafness pathogenic genes. First, the fragment DNA is cyclized. As long as the cleavage point is not in the template of the backward extended primers, the amplification can be conducted. Thus, the adaptation range of the primer amplification and the effective amount of templates are increased, and the detection sensitivity of the fragment DNA is also improved dramatically. After connection of the plasma DNA and linkers, the pre-library is amplified and is subsequently cyclized. Cyclization of the pre-library can be auto-connection to a ring. The invention selects single strand splint cyclization with specific auxiliary sequences. Preferably, the linkers connected to plasma fragment DNA are partially matched Y-type linkers, wherein the length of matched base that next to barcode is 9bp, and the length of unmatched base is 7bp. Preferably, a single strand DNA that is complementary to the Y-type linkers is used in assisting the cyclization. The linkers and auxiliary sequences are complementary to each other to form double strands, wherein the length of the auxiliary sequence is 32bp. Preferably, the ends between the linker sequences are connected by Taq DNA Ligase (M0208L) from NEB.

### Auxiliary sequence:

Bridge (SEQ ID NO: 31): GTGCGTCCCTACTCTGTGTGCATGGGACGCAC

### Specific Embodiments

### Experiment protocols:

### 1. Extraction of plasma DNA

Plasma DNA was extracted from 1-2ml plasma by QIAamp Circulating Nucleic Acid Kit (CAT No. 55114). DNA was eluted in 45µl elution buffer, wherein 2µl is used for concentration detection by Qubit.

### 2. End-filing and addition of A on the plasma DNA

The reaction mixture was prepared as shown in Table 1.

**Table 1**

| | |
|---|---|
| T4 DNA polymerase buffer (10 X) | 5 µl |
| Plasma DNA | 40.5 µl |
| Taq polymerase | 0.5 µl |
| T4 DNA polymerase | 2.0 µl |
| 10 mM dNTP | 2.0 µl |
| Total volume | 50 µl |

### Reaction on a PCR machine:

37°C: 20 min
72°C: 20 min
4°C: maintain

The product with A addition was purified on a column, dissolved in 25 µl Buffer EB, and eluted twice.

### 3. Connection with the linkers

The reaction mixture was prepared as shown in Table 2.

**Table 2**

| | |
|---|---|
| DNA | 22 µl |
| 2X Quick Ligase Buffer | 25 µl |
| 7.5 µM CycAB linker | 2 µl |
| T4 DNA ligase (HC) | 1 µl |
| Total volume | 50 µl |

### Reaction on a PCR machine:

20°C: 15 min
65°C: 10 min
4°C: maintain

### 4. Construction of pre-library

### 4.1 PCR (100 µl system), the reaction mixture was prepared as shown in Table 3.

**Table 3**

| | |
|---|---|
| Phusion PCR Master Mix (2X) | 50 µl |
| CycUniprimer (F-10 µM, R-35 µM) | 2 µl |
| Products connected with linkers | 50 µl |
| Total volume | 100 µl |

### 4.2 PCR programs are shown in Table 4.

**Table 4**

| | | |
|---|---|---|
| 98°C | 30s | 1 cycle |
| 98°C | 10s | 14 cycles |
| 65°C | 30s | |
| 72°C | 30s | |
| 72°C | 5 min | 1 cycle |
| 4°C | maintain | |

### 5. Phosphorylation and cyclization

### 5.1 The phosphorylation and cyclization system was prepared as shown in Table 5.

**Table 5**

| | |
|---|---|
| ATP | 0.4 µl |
| T4 PNK | 0.5 µl |
| 10X Taq Ligase Buffer | 4 µl |
| Bridge (10 µM) | 4 µl |
| Pre-library products | 12 µl |
| Taq Ligase | 2 µl |
| EB | 17.1 µl |
| Total volume | 50 µl |

### 5.2 5.2 PCR programs are shown in Table 6.

**Table 6**

| | | |
|---|---|---|
| 37°C | 30 min | |
| 95°C | 30s | 30 cycles |
| 50°C | 2 min | |
| 4°C | maintain | |

### 6. Exonuclease digestion

### 6.1 components as shown in Table 7 were added in the reaction system of step 5 in order.

**Table 7**

| | |
|---|---|
| Exo I | 1 µl |
| Exo III | 1 µl |
| Reaction on a PCR machine at 37°C for 10 min | |
| PK (3 mg/ml) | 1 µl |

### 6.2 Reaction conditions are as shown in Table 8.

**Table 8**

| | |
|---|---|
| 50°C | 10 min |
| 99°C | 4 min |
| 4°C | maintain |

### 7. PCR screening of the target region using the backward extended primers

The detection of the 22 loci of GJB2 gene, GJB3 gene and SLC26A4 gene was performed in one PCR using 9 pairs of primers by means of Multi-PCR, which solve multi-point detection.

### 7.1 PCR reaction system was prepared as shown in Table 9

**Table 9**

| | |
|---|---|
| Phusion PCR Master Mix (2x) | 50 µl |
| Primer Mix (0.5-2 µM for each) | 4 µl |
| Cyclized DNA | 43 µl |
| EB | 3 µl |
| Total volume | 50 µl |

### 7.2 PCR reaction conditions are as shown in Table 10

**Table 10**

| | | |
|---|---|---|
| 98°C | 30s | 1 cycle |
| 98°C | 10s | 25 cycles |
| 60°C | 30s | |
| 72°C | 30s | |
| 72°C | 5 min | 1 cycle |
| 4°C | maintain | |

After PCR reaction, the product was immediately purified using 90 µl XP Beads (0.9x), and then dissolved in 26 µl Buffer EB.

### 8. Generation of the final library

### 8.1 PCR reaction system was prepared as shown in Table 11.

**Table 11**

| | |
|---|---|
| Phusion PCR Master Mix (2x) | 25 µl |
| Illumina-Nextera-F (25 µM) | 0.5 µl |
| Index Primer (25 µM) | 0.5 µl |
| Screened PCR products | 24 µl |
| Total volume | 50 µl |

### 8.2 PCR reaction conditions were as shown in Table 12

**Table 12**

| | | |
|---|---|---|
| 98°C | 30s | 1 cycle |
| 98°C | 10s | 25 cycles |
| 65°C | 30s | |
| 72°C | 30s | |
| 72°C | 5 min | 1 cycle |
| 4°C | maintain | |

After PCR reaction, 10 µl product was analysed by electrophoresis on 2% agarose gel, while other products were purified and recycled using 0.8x XP Beads, and were finally dissolved in 22 µl EB Buffer, which were used in a subsequent Q-PCR as the final library. The size of the final library is 320 bp (used in calculating QPCR concentration), and it should mix with Read1 Sequencing Primer from NEXTERA for sequencing. 300bp double-end sequencing was performed using Miseq from Illumina.

The sample was plasma from a pregnant woman. Preferably, the mutation of the pre-determined loci is at least one gene mutation selected from the 22 loci of GJB2 gene, GJB3 gene, and SLC26A4 gene. More preferably, the mutation of GJB2 gene is at least one mutation selected from 35delG, 109G>A(VAL37ILE)167delT, 176-191dell6, 235delC and 299_300delAT; the mutation of GJB3 gene is at least one mutation selected from 421A>G(ILE141VAL), 421-423delATT, 497A-G(ASN166SER), 538C>T(ARG180TER), 547G>A(GLU183LYS) and 580G>A(ALA194THR); and the mutation of SCL26A4 gene is at least one mutation selected from 281C>T, 589G>A, IVS7-2A>G, 1174A>T(N392Y), 1226G>A, 1229C>T(T410M), 1975G>C, 2027T>A(L676Q), 2162C>T(T721M), 2168A>G(HIS723ARG) and IVS15+5G>A. For convenience of description, the above mutation loci tested by primer combinations are summarized as shown in Table 13.

**Table 13 Primers and information of the pre-determined loci to be tested**

| Gene | Primers corresponding to the tested locus | locus | HGMD observation |
|---|---|---|---|
| GJB2 | GJB2-F1: | 35delG | CD972240 deafness, autosomal recessive inheritance |
| | CACGCTGCAGACGATCC | | |
| | GJB2-R1: | | |
| | CCCCAATCCATCTTCTACTCT | | |
| | | 109G>A (VAL37ILE) | CM000016 deafness, autosomal recessive inheritance |
| | GJB2-F2: | 167delT | CD972241 deafness, autosomal recessive inheritance |
| | TCCCACATCCGGCTATG | 176-191del16 | CD000073 deafness, autosomal recessive inheritance |
| | GJB2-R: | | |
| | | 235delC | CD991730 deafness, autosomal recessive inheritance |
| | | 299-300delAT | CD000074 deafness, autosomal recessive inheritance |
| GJB3 | | 421A>G (ILE141VAL) | CM000019 deafness, autosomal recessive inheritance |
| | GJB3-F: | 421-423delATT | CD000075 deafness, autosomal recessive inheritance |
| | CGTGGACTGCTACATTGCC | 497A-G (ASN166SER) | CM090826 deafness, non-syndrome, autosomal recessive inheritance |
| | GJB3-R: | | |
| | ATGTTGGGGCAGGGG | 538C>T (ARG180TER) | CM980934 deafness, non-syndrome, autosomal dominant |
| | | 547G>A (GLU183LYS) | CM980935 deafness, non-syndrome, autosomal dominant |
| | | 580G>A (ALA194THR) | CM090827 deafness, non-syndrome, autosomal recessive inheritance |
| | PDS2-F: | 281C>T | CM074541 LVAS |
| | CGTCATTTCGGGAGT | | |
| | PDS2-R: | | |
| | CTAAGCAGCCATTC | | |
| | PDS5-F: | 589G>A | CM074557 LVAS |
| | CCCTGACTCTGCTGG | | |
| | PDS5-R: | | |
| | CACTGGCAATCAGGA | | |
| | PDS7-F2: | IVS7-2 A>G | CS991479 deafness syndrome Pendred syndrome |
| | TGGCAGTAGCAATTATCGTC | | |
| | PDS7-R2: | | |
| | TTTCATATGGAGCCAACCTG | | |
| | PDS10-F: | 1174 A>T (N392Y) | CM030959 deafness, non-syndrome, autosomal recessive inheritance |
| | CCACTGCTCTTTCCCGC | | |
| | PDS10-R: | | |
| SCL26A4 | | 1226G>A | CM981503 deafness syndrome |
| | | 1229 C>T(T410M) | CM981504 deafness syndrome |
| | PDS17-F: | 1975G>C | CM073354 deafness, non-syndrome, autosomal recessive inheritance |
| | TTCCTGGACGTTGTTGGAG | | |
| | PDS17-R: | | |
| | | 2027 T>A (L676Q) | CM030963 deafness, non-syndrome, autosomal recessive inheritance |
| | PDS19-F: | 2162C>T (T721M) | CM991031 deafness, non-syndrome, autosomal recessive inheritance |
| | TCTTGAGATTTCACTTGGTT | | |
| | PDS19-R: | 2168 A>G (HIS723ARG) | CM981513 deafness syndrome |
| | GTTCCATTTTAGAAACGGTA | IVS15+5G> A | CS050413 LVAS |

### Experiment Results:

**Table 14**

| Sample | Information provided by hospital | | Results of non-invasive detection | | | | Conclusion of non-invasive detection | |
|---|---|---|---|---|---|---|---|---|
| | Maternal genotype | Fetal genotype | mutation | mutated templates¹ | Total templates² | % | Maternal genotype | Fetal genotype |
| Pregnant woman 1 | No mutation was observed. | GJB2 109 G>A heterologous mutation | GJB2 109 G>A | 16 | 331 | 4.83% | No mutation was observed. | GJB2 109G>A fetus is heterologous. |
| | | | SLC26A4 IVS7-2A> G | 0 | 376 | 0.00% | | |
| | | | GJB2 299-300delAT | 0 | 162 | 0.00% | | |
| | | | GJB3 538C>T | 0 | 262 | 0.00% | | |
| Pregnant woman 2 | GJB2 109 G>A heterologous mutation | GJB2 109 G>A heterologous mutation | GJB2 109 G>A | 78 | 159 | 49.06 % | GJB2 109 G>A Mother is heterologous. | GJB2 109G>A fetus is heterologous. |
| | | | SLC26A4 IVS7-2A>G | 0 | 198 | 0.00% | | |
| | | | GJB2 299-300delAT | 0 | 72 | 0.00% | | |
| | | | GJB3 538C>T | 0 | 162 | 0.00% | | |
| Pregnant woman 3 | GJB2 109 G>A heterologous mutation | GJB2 109 G>A heterologous mutation | GJB2 109 G>A | 177 | 343 | 51.60 % | GJB2 109 G>A Mother is heterologous. | GJB2 109 G>A fetus is heterologous. |
| | | | SLC26A4 IVS7-2A>G | 0 | 425 | 0.00% | | |
| | | | GJB2 299-300delAT | 0 | 132 | 0.00% | | |
| | | | GJB3 538C>T | 0 | 362 | 0.00% | | |
| Pregnant woman 4 | GJB2 109 G>A heterologous mutation | GJB2 109 G>A Homozygous mutation | GJB2 109 G>A | 52 | 93 | 55.91 % | GJB2 109 G>A Mother is heterologous. | GJB2 109 G>A Fetus is homozygous. |
| | | | SLC26A4 IVS7-2A>G | 0 | 137 | 0.00% | | |
| | | | GJB2 299-300del AT | 0 | 41 | 0.00% | | |
| | | | GJB3 538C>T | 1 | 97 | 1.03% | | |
| Pregnant woman 5 | SLC26A4 IVS7-2I heterologous mutation | SLC26A4 IVS7-2 heterologous mutation | GJB2 109 G>A | 0 | 67 | 0.00% | SLC26A4 IVS7-2A>G Mother is heterologous. | SLC26A4 IVS7-2A>G fetus is heterologous. |
| | | | SLC26A4 IVS7-2A>G | 59 | 114 | 51.75 % | | |
| | | | GJB2 299-300delAT | 0 | 28 | 0.00% | | |
| | | | GJB3 538C>T | 0 | 71 | 0.00% | | |
| Pregnant woman 6 | GJB2 109 G>A heterologous mutation | No mutation was observed. | GJB2 109 G>A | 383 | 837 | 45.76 % | GJB2 109 G>A Mother is heterologous. | No mutation was observed. |
| | | | SLC26A4 IVS7-2A>G | 0 | 958 | 0.00% | | |
| | | | GJB2 299-300delAT | 0 | 381 | 0.00% | | |
| | | | GJB3 538C>T | 0 | 702 | 0.00% | | |
| Pregnant woman 7 | No mutation was observed. | GJB2 109 G>A heterologous mutation | GJB2 109 G>A | 10 | 122 | 8.20% | No mutation was observed. | GJB2 109 G>A fetus is heterologous. |
| | | | SLC26A4 IVS7-2A>G | 0 | 157 | 0.00% | | |
| | | | GJB2 299-300delAT | 0 | 43 | 0.00% | | |
| | | | GJB3 538C>T | 1 | 107 | 0.93% | | |
| Pregnant woman 8 | No mutation was observed. | GJB2 299-300delAT heterologous mutation | GJB2 109 G>A | 0 | 291 | 0.00% | No mutation was observed. | GJB2 299-300delAT fetus is heterologous. |
| | | | SLC26A4 IVS7-2A>G | 0 | 381 | 0.00% | | |
| | | | GJB2 299-300delAT | 14 | 149 | 9.40% | | |
| | | | GJB3 538C>T | 0 | 255 | 0.00% | | |
| Pregnant woman 9 | No mutation was observed. | GJB2 109 G>A heterologous mutation | GJB2 109 G>A | 34 | 627 | 5.42% | No mutation was observed. | GJB2 109 G>A fetus is heterologous. |
| | | | SLC26A4 IVS7-2A>G | 0 | 748 | 0.00% | | |
| | | | GJB2 299-300delAT | 0 | 271 | 0.00% | | |
| | | | GJB3 538C>T | 0 | 506 | 0.00% | | |
| Pregnant woman 10 | GJB2 109 G>A heterologous mutation | No mutation was observed. | GJB2 109 G>A | 79 | 169 | 46.75% | GJB2 109 G>A Mother is heterologous. | No mutation was observed. |
| | | | SLC26A4 IVS7-2A>G | 0 | 250 | 0.00% | | |
| | | | GJB2 299-300delAT | 0 | 75 | 0.00% | | |
| | | | GJB3 538C>T | 0 | 188 | 0.00% | | |
| Pregnant woman 11 | GJB2 109 G>A heterologous mutation | GJB2 109 G>A heterologous mutation | GJB2 109 G>A | 117 | 233 | 50.21 % | GJB2 109 G>A Mother is heterologous. | GJB2 109 G>A fetus is heterologous. |
| | | | SLC26A4 IVS7-2A>G | 0 | 265 | 0.00% | | |
| | | | GJB2 299-300delAT | 0 | 83 | 0.00% | | |
| | | | GJB3 538C>T | 0 | 205 | 0.00% | | |
| Pregnant woman 12 | GJB3 538C>T heterologous mutation | No mutation was observed. | GJB2 109 G>A | 0 | 287 | 0.00% | GJB3 538C>T Mother is heterologous. | No mutation was observed. |
| | | | SLC26A4 IVS7-2A>G | 0 | 354 | 0.00% | | |
| | | | GJB2 299-300delAT | 0 | 130 | 0.00% | | |
| | | | GJB3 538C>T | 124 | 292 | 42.47 % | | |
| Pregnant woman 13 | No mutation was observed. | No mutation was observed. | GJB2 109 G>A | 0 | 465 | 0.00% | No mutation was observed. | No mutation was observed. |
| | | | SLC26A4 IVS7-2A> G | 0 | 577 | 0.00% | | |
| | | | GJB2 299-300delAT | 0 | 231 | 0.00% | | |
| | | | GJB3 538C>T | 0 | 447 | 0.00% | | |
| Pregnant woman 14 | No mutation was observed. | No mutation was observed. | GJB2 109 G>A | 0 | 388 | 0.00% | No mutation was observed. | No mutation was observed. |
| | | | SLC26A4 IVS7-2A>G | 0 | 470 | 0.00% | | |
| | | | GJB2 299-300delAT | 0 | 176 | 0.00% | | |
| | | | GJB3 538C>T | 2 | 313 | 0.64% | | |
| Pregnant woman 15 | No mutation was observed. | No mutation was observed. | GJB2 109 G>A | 0 | 248 | 0.00% | No mutation was observed. | No mutation was observed. |
| | | | SLC26A4 IVS7-2A> G | 0 | 283 | 0.00% | | |
| | | | GJB2 299-300delAT | 0 | 82 | 0.00% | | |
| | | | GJB3 538C>T | 1 | 219 | 0.46% | | |
| Pregnant woman 16 | No mutation was observed. | No mutation was observed. | GJB2 109 G>A | 0 | 84 | 0.00% | No mutation was observed. | No mutation was observed. |
| | | | SLC26A4 IVS7-2A>G | 0 | 94 | 0.00% | | |
| | | | GJB2 299-300delAT | 0 | 35 | 0.00% | | |
| | | | GJB3 538C>T | 0 | 64 | 0.00% | | |
| Pregnant woman 17 | No mutation was observed. | No mutation was observed. | GJB2 109 G>A | 0 | 275 | 0.00% | No mutation was observed. | No mutation was observed. |
| | | | SLC26A4 IVS7-2A>G | 0 | 380 | 0.00% | | |
| | | | GJB2 299-300delAT | 0 | 86 | 0.00% | | |
| | | | GJB3 538C>T | 0 | 304 | 0.00% | | |
| Pregnant woman 18 | No mutation was observed. | No mutation was observed. | GJB2 109 G>A | 0 | 364 | 0.00% | No mutation was observed. | No mutation was observed. |
| | | | SLC26A4 IVS7-2A>G | 0 | 481 | 0.00% | | |
| | | | GJB2 299-300delAT | 0 | 163 | 0.00% | | |
| | | | GJB3 538C>T | 0 | 385 | 0.00% | | |

| | | | | | | | | |
|---|---|---|---|---|---|---|---|---|
| Notes: 1. Mutated templates: the number of templates of the corresponding mutated loci in the non-invasive prenatal detection. 2. Total templates: the total number of templates of the corresponding loci in the non-invasive prenatal detection. | | | | | | | | |

Main reagents include: QIAamp Circulating Nucleic Acid Kit, T4 DNA phosphorylation buffer (10x), 10 µM dNTP mixture, T4 DNA polymerase, T4 DNA phosphorylase, dATP solution, Quick ligation buffer(5x), Y-type DNA double strand linkers, T4 DNA Ligase(HC), Quick T4 DNA ligase (NEB), Phusion DNA polymerase (Phusion DNA polymerase mixture), pre-amplification primers, Ultra-pure water, 10x Taq ligase Buffer, Taq ligase, 10x NEBuffer 1, Exo III.

### The data standard for the conclusion of the experiment results:

According to an internal statistics, the average amount of fetal plasma DNA in a pregnant woman at gestational age of 12-26 weeks is around 10% based on the amount of plasma DNA of the pregnant woman. Deafness gene disorder is autosomal inherited, and thus the data standard of various combinations of maternal and fetal pathogenic genotypes can be obtained based on the laws of inheritance. The experiment conclusion is deduced based on the genotype data of the tested pathogenic genotypes.

| Genotypes | | Theoretical values | | |
|---|---|---|---|---|
| Mother | Fetus | mother | fetus | total |
| Homozygous mutation | Heterologous mutation | 90% | 10% | 100% |
| Homozygous mutation | Heterologous mutation | 90% | 5% | 95% |
| Heterologous mutation | Homozygous mutation | 45% | 10% | 55% |
| Heterologous mutation | Heterologous mutation | 45% | 5% | 50% |
| Heterologous mutation | Wild type | 45% | 0% | 45% |
| Wild type | Heterologous mutation | 0% | 5% | 5% |
| Wild type | Wild type | 0% | 0% | 0% |

## Claims

1. A kit for non-invasively detecting fetal deafness pathogenic gene mutations, comprising: reagents for extracting plasma DNAs, barcode linker for labeling the extracted plasma DNAs through the start and terminal positions of the extracted plasma DNAs and 6 different bases on 3' end of the barcode linker, a DNA cyclase, primers and reagents for amplifying target DNAs, and primers and reagents for pre-amplifying the pre-determined loci of deafness pathogenic genes, wherein the primers for pre-amplifying the pre-determined loci of deafness pathogenic genes are pairs of primers that are backward extended and designed to simultaneously testing a plurality of mutation loci comprising 22 loci of GJB2 gene, GJB3 gene, and SLC26A4 gene; the pairs of primers being:
GJB2-F1 (SEQ ID NO: 2): CACGCTGCAGACGATCC,
GJB2-R1 (SEQ ID NO: 3): CCCCAATCCATCTTCTACTCT;
GJB2-F2 (SEQ ID NO: 4): TCCCACATCCGGCTATG,
GJB2-R2 (SEQ ID NO: 5): GATGGGGAAGTAGTGATCGTAG;
GJB3-F (SEQ ID NO: 7): CGTGGACTGCTACATTGCC,
GJB3-R (SEQ ID NO: 8): ATGTTGGGGCAGGGG;
PDS3-F (SEQ ID NO: 10): CGTCATTTCGGGAGT,
PDS3-R (SEQ ID NO: 11): CTAAGCAGCCATTCC;
PDS5-F (SEQ ID NO: 13): CCCTGACTCTGCTGG,
PDS5-R (SEQ ID NO: 14): CACTGGCAATCAGGA;
PDS7-F2 (SEQ ID NO: 16): TGGCAGTAGCAATTATCGTC,
PDS7-R2 (SEQ ID NO: 17): TTTCATATGGAGCCAACCTG;
PDS10-F (SEQ ID NO: 19): CCACTGCTCTTTCCCGC,
PDS10-R (SEQ ID NO: 20): CAAGAGAAGAATCCTGAGAAGATG;
PDS17-F (SEQ ID NO: 22): TTCCTGGACGTTGTTGGAG,
PDS17-R (SEQ ID NO: 23): GATATAGCTCCACAGTCAAGCAC; and
PDS19-F (SEQ ID NO: 25): TCTTGAGATTTCACTTGGTT,
PDS19-R (SEQ ID NO: 26): GTTCCATTTTAGAAACGGTA.

2. The kit according to claim 1, **characterized in that** the kit further comprises reagents for high throughput sequencing.

3. The kit according to claim 1, **characterized in that** the backward extended pair of primers contain universal primer region suitable for different high-throughput sequencing platforms.

4. The kit according to claim 1, **characterized in that** there are at least two base differences between the linkers.

5. The kit according to claim 1, **characterized in that** the linkers are partially matched Y-type linkers.

6. The kit according to claim 1, **characterized in that** the deafness pathogenic genes have insertion, deletion, substitution or gene fusion mutations.

7. A set of primer pairs as defined in claim 1 for the use of non-invasively detecting deafness pathogenic gene mutations in a fetus.

8. A method for non-invasive detection of fetal deafness pathogenic gene mutations, comprising:
--designing primers according to a plurality of pre-determined mutation loci of deafness pathogenic genes comprising 22 loci of GJB2 gene, GJB3 gene and SLC26A4 gene;
--simultaneously testing the plurality of pre-determined mutation loci of deafness pathogenic genes by:
----connecting plasma DNAs contained in a plasma DNA sample obtained from a pregnant woman with pre-amplification barcode linkers to obtain connected products by labeling the start and terminal positions of the plasma DNAs and 6 different bases on 3' end of the pre-amplification barcode linker;
----PCR-pre-amplifying the connected product to obtain pre-amplified products;
----cyclizing the pre-amplified product to obtain cyclized DNAs;
----PCR amplifying the cyclized DNAs using the designed primers to obtain amplified products; and
----high throughput sequencing the amplified products and detecting mutations of the fetal deafness pathogenic genes wherein the primers are a set of pairs of primers that are backward extended and are:
GJB2-F1 (SEQ ID NO: 2): CACGCTGCAGACGATCC,
GJB2-R1 (SEQIDNO: 3): CCCCAATCCATCTTCTACTCT;
GJB2-F2 (SEQ ID NO: 4): TCCCACATCCGGCTATG,
GJB2-R2 (SEQIDNO: 5): GATGGGGAAGTAGTGATCGTAG;
GJB3-F (SEQ ID NO: 7): CGTGGACTGCTACATTGCC,
GJB3-R (SEQ ID NO: 8): ATGTTGGGGCAGGGG;
PDS3-F (SEQ ID NO: 10): CGTCATTTCGGGAGT,
PDS3-R (SEQIDNO: 11): CTAAGCAGCCATTCC;
PDS5-F (SEQIDNO: 13): CCCTGACTCTGCTGG,
PDS5-R (SEQ ID NO : 14): CACTGGCAATCAGGA;
PDS7-F2 (SEQ ID NO: 16): TGGCAGTAGCAATTATCGTC,
PDS7-R2 (SEQ ID NO: 17): TTTCATATGGAGCCAACCTG;
PDS10-F (SEQ ID NO : 19): CCACTGCTCTTTCCCGC,
PDS10-R (SEQ ID NO : 20): CAAGAGAAGAATCCTGAGAAGATG;
PDS17-F (SEQ ID NO : 22): TTCCTGGACGTTGTTGGAG,
PDS17-R (SEQ ID NO : 23): GATATAGCTCCACAGTCAAGCAC; and
PDS19-F (SEQ ID NO : 25): TCTTGAGATTTCACTTGGTT,
PDS19-R (SEQ ID NO : 26): GTTCCATTTTAGAAACGGTA.

9. The method of claim 8 wherein the at least one backward extended pair of primers contains universal primer region suitable for different high-throughput sequencing platforms.

10. The method of claim 8, wherein there are at least two base differences between the linkers.

11. The method of any one of claims 8 and 9, wherein the linkers are partially matched Y-type linkers.

12. The method of any one of claims 8 to 11, wherein cyclizing the pre-amplified products comprises a splint cyclization, wherein single stand DNAs complementary to both ends of the pre-amplified DNA are used as splints; and closing the ring is completed by a heat-resistant Taq ligase.

13. The method of any one of claims 8 to 12, wherein cyclizing the pre-amplified products comprises multiple reactions of a single system circulation consisting of DNA denaturation, splint DNA annealing and connecting.

14. The method of any one of claims 8 to 13, further comprising digesting the uncyclized linear DNA.

15. The method of any one of claims 8 to 14, wherein the deafness pathogenic gene mutations comprise at least one of insertion, deletion, substitution or gene fusion mutations.

## Patentansprüche

1. Kit zum nicht-invasiven Nachweisen von pathogenen Genmutationen der fötalen Gehörlosigkeit, umfassend: Reagenzien zur Extraktion von Plasma-DNAs, Barcode-Linker zum Kennzeichnen der extrahierten Plasma-DNAs über die Start- und Terminalpositionen der extrahierten Plasma-DNAs und 6 unterschiedliche Basen am 3' Ende des Barcode-Linkers, eine DNA-Cyclase, Primer und Reagenzien zum Amplifizieren von Ziel-DNAs, und Primer und Reagenzien zum Voramplifizieren der vorbestimmten Loci von pathogenen Genen der Gehörlosigkeit, wobei die Primer zum Voramplifizieren der vorbestimmten Loci von pathogenen Genen der Gehörlosigkeit Primer-Paare sind, die rückwärts verlängert und vorgesehen sind, um gleichzeitig eine Vielzahl von Mutations-Loci zu testen, die 22 Loci des GJB2 Gens, GJB3 Gens und SLC26A4 Gens umfassen, wobei die Primer-Paare sind:
GJB2-F1 (SEQ ID Nr: 2): CACGCTGCAGACGATCC
GJB2-R1 (SEQ ID Nr: 3): CCCCAATCCATCTTCTACTCT
GJB2-F2 (SEQ ID Nr: 4): TCCCACATCCGGCTATG
GJB2-R2 (SEQ ID Nr: 5): GATGGGGAAGTAGTGATCGTAG
GJB3-F (SEQ ID Nr: 7): CGTGGACTGCTACATTGCC
GJB3-R (SEQ ID Nr: 8): ATGTTGGGGCAGGGG
PDS3-F (SEQ ID Nr: 10): CGTCATTTCGGGAGT
PDS3-R (SEQ ID Nr: 11): CTAAGCAGCCATTCC
PDS5-F (SEQ ID Nr: 13): CCCTGACTCTGCTGG
PDS5-R (SEQ ID Nr: 14): CACTGGCAATCAGGA
PDS7-F2 (SEQ ID Nr: 16): TGGCAGTAGCAATTATCGTC
PDS7-R2 (SEQ ID Nr: 17): TTTCATATGGAGCCAACCTG
PDS10-F (SEQ ID Nr: 19): CCACTGCTCTTTCCCGC
PDS10-R (SEQ ID Nr: 20): CAAGAGAAGAATCCTGAGAAGATG
PDS17-F (SEQ ID Nr: 22): TTCCTGGACGTTGTTGGAG
PDS17-R (SEQ ID Nr: 23): GATATAGCTCCACAGTCAAGCAC
PDS19-F (SEQ ID Nr: 25): TCTTGAGATTTCACTTGGTT
PDS19-R (SEQ ID Nr: 26): GTTCCATTTTAGAAACGGTA

2. Kit nach Anspruch 1, **dadurch gekennzeichnet, dass** der Kit ferner Reagenzien für die Hochdurchsatz-Sequenzierung umfasst.

3. Kit nach Anspruch 1, **dadurch gekennzeichnet, dass** das rückwärts verlängerte Primer-Paar eine universelle Primer-Region, geeignet für unterschiedliche Hochdurchsatz-Sequenzierungs-Plattformen, umfasst.

4. Kit nach Anspruch 1, **dadurch gekennzeichnet, dass** zwischen den Linkern mindestens zwei Basen-Unterschiede bestehen.

5. Kit nach Anspruch 1, **dadurch gekennzeichnet, dass** die Linker teilweise passende Y-Linker sind.

6. Kit nach Anspruch 1, **dadurch gekennzeichnet, dass** die pathogenen Gene der Gehörlosigkeit Insertions-, Deletions-, Substitutions- oder Genfusionsmutationen aufweisen.

7. Satz Primer-Paare wie in Anspruch 1 definiert zur Verwendung eines nichtinvasiven Nachweises von pathogenen Genmutationen der Gehörlosigkeit bei einem Fötus.

8. Verfahren zum nicht-invasiven Nachweisen von pathogenen Genmutationen der fötalen Gehörlosigkeit, umfassend:
- Entwerfen von Primern nach einer Vielzahl von vorbestimmten Mutations-Loci von pathogenen Genen der Gehörlosigkeit, umfassend 22 Loci des GJB2 Gens, GJB3 Gens und SLC26A4 Gens;
- gleichzeitiges Testen der Vielzahl von vorbestimmten Mutations-Loci von pathogenen Genen der Gehörlosigkeit durch;
- Verbinden von Plasma-DNAs, die in einer Plasma-DNA-Probe, erhalten von einer schwangeren Frau, enthalten sind, mit Voramplifikations-Barocde-Linkern, um verbundene Produkte durch Kennzeichnen der Start- und Terminalpositionen der Plasma-DNAs und 6 unterschiedlichen Basen am 3' Ende des voramplifizierten Barcode-Linkers zu erhalten;
- PCR-Voramplifizieren des verbundenen Produkts, um voramplifizierte Produkte zu erhalten;
- Zyklisieren des voramplifizierten Produkts, um zyklisierte DNAs zu erhalten;
- PCR-Amplifizieren der zyklisierten DNAs unter Verwendung der entworfenen Primer, um amplifizierte Produkte zu erhalten; und
- Hochdurchsatz-Sequenzieren der amplifizierten Produkte und Nachweisen von pathogenen Genmutationen der fötalen Gehörlosigkeit,
wobei die Primer ein Satz Primer-Paare sind, die rückwärts verlängert sind, und sind:
GJB2-F1 (SEQ ID Nr: 2): CACGCTGCAGACGATCC
GJB2-R1 (SEQ ID Nr: 3): CCCCAATCCATCTTCTACTCT
GJB2-F2 (SEQ ID Nr: 4): TCCCACATCCGGCTATG
GJB2-R2 (SEQ ID Nr: 5): GATGGGGAAGTAGTGATCGTAG
GJB3-F (SEQ ID Nr: 7): CGTGGACTGCTACATTGCC
GJB3-R (SEQ ID Nr: 8): ATGTTGGGGCAGGGG
PDS3-F (SEQ ID Nr: 10): CGTCATTTCGGGAGT
PDS3-R (SEQ ID Nr: 11): CTAAGCAGCCATTCC
PDS5-F (SEQ ID Nr: 13): CCCTGACTCTGCTGG
PDS5-R (SEQ ID Nr: 14): CACTGGCAATCAGGA
PDS7-F2 (SEQ ID Nr: 16): TGGCAGTAGCAATTATCGTC
PDS7-R2 (SEQ ID Nr: 17): TTTCATATGGAGCCAACCTG
PDS10-F (SEQ ID Nr: 19): CCACTGCTCTTTCCCGC
PDS10-R (SEQ ID Nr: 20): CAAGAGAAGAATCCTGAGAAGATG
PDS17-F (SEQ ID Nr: 22): TTCCTGGACGTTGTTGGAG
PDS17-R (SEQ ID Nr: 23): GATATAGCTCCACAGTCAAGCAC
PDS19-F (SEQ ID Nr: 25): TCTTGAGATTTCACTTGGTT
PDS19-R (SEQ ID Nr: 26): GTTCCATTTTAGAAACGGTA

9. Verfahren nach Anspruch 8, wobei das mindestens eine, rückwärts erweiterte Primer-Paar eine universelle Primer-Region enthält, die für unterschiedliche Hochdurchsatz-Sequenzierungplattformen geeignet ist.

10. Verfahren nach Anspruch 8, wobei mindestens zwei Basen-Unterschiede zwischen den Linkern bestehen.

11. Verfahren nach einem der Ansprüche 8 und 9, wobei die Linker teilweise passende Y-Linker sind.

12. Verfahren nach einem der Ansprüche 8 bis 11, wobei Zyklisieren der voramplifizierten Produkte eine Splint-Zyklisierung umfasst, wobei Einzelstrang-DNAs, die beiden Enden der voramplifizierten DNA entsprechen, als Splints verwendet werden, und Schließen des Rings durch eine Hitze-resistente Taq-Ligase vervollständigt wird.

13. Verfahren nach einem der Ansprüche 8 bis 12, wobei Zyklisieren der voramplifizierten Produkte mehrfache Reaktionen eines zirkulierenden Einzelsystems, das aus DNA-Denaturierung, Splint-DNA-Annealing und DNA-Verbinden besteht, umfasst.

14. Verfahren nach einem der Ansprüche 8 bis 13, ferner umfassend Verdauen der nicht zyklisierten, linearen DNA.

15. Verfahren nach einem der Ansprüche 8 bis 14, wobei die pathogenen Genmutationen der Gehörlosigkeit mindestens eine von Insertions-, Deletions-, Subsitutions- oder Genmutationen umfassen.

## Revendications

1. Kit pour détecter de manière non invasive des mutations de gènes pathogènes de surdité fœtale, comprenant : des réactifs pour extraire des ADN plasmatiques, un lieur à code à barres pour marquer les ADN plasmatiques extraits par l'intermédiaire des positions de début et terminales des ADN plasmatiques extraits et de 6 bases différentes sur l'extrémité 3' du lieur à code à barres, une ADN cyclase, des amorces et des réactifs pour amplifier des ADN cibles, et des amorces et des réactifs pour pré-amplifier les locus prédéterminés de gènes pathogènes de surdité, dans lequel les amorces pour pré-amplifier les locus prédéterminés de gènes pathogènes de surdité sont des paires d'amorces qui sont prolongées vers l'arrière et conçues pour tester simultanément une pluralité de locus de mutation comprenant 22 locus de gène GJB2, de gène GJB3, et de gène SLC26A4 ;
les paires d'amorces étant :
GJB2-F1 (SEQ ID NO: 2): CACGCTGCAGACGATCC,
GJB2-R1 (SEQ ID NO : 3): CCCCAATCCATCTTCTACTCT;
GJB2-F2 (SEQ ID NO: 4); TCCCACATCCGGCTATG,
GJB2-R2 (SEQ ID NO: 5): GATGGGGAAGTAGTGATCGTAG;
GJB3-F (SEQ ID NO: 7): CGTGGACTGCTACATTGCC,
GJB3-R (SEQ ID NO: 8): ATGTTGGGGCAGGGG;
PDS3-F (SEQ ID NO: 10): CGTCATTTCGGGAGT,
PDS3-R (SEQ ID NO: 11). CTAAGCAGCCATTCC;
PDS5-F (SEQ ID NO: 13): CCCTGACTCTGCTGG,
PDS5-R (SEQ ID NO; 14); CACTGGCAATCAGGA;
PDS7-F2 (SEQ ID NO: 16): TGGCAGTAGCAATTATCGTC,
PDS7-R2 (SEQ ID NO : 17): TTTCATATGGAGCCAACCTG;
PDS10-F (SEQ ID NO: 19): CCACTGCTCTTTCCCGC,
PDS10-R (SEQ ID NO: 20): CAAGAGAAGAATCCTGAGAAGATG:
PDS17-F (SEQ ID NO: 22): TTCCTGGACGTTGTTGGAG,
PDS17-R (SEQ ID NO : 23) : GATATAGCTCCACAGTCAAGCAC; et
PDS19-F (SEQ ID NO: 25); TCTTGAGATTTCACTTGGTT,
PDS19-R (SEQ ID NO: 26): GTTCCATTTTAGAAACGGTA.

2. Kit selon la revendication 1, **caractérisé en ce que** le kit comprend en outre des réactifs pour un séquençage à haut débit.

3. Kit selon la revendication 1, **caractérisé en ce que** la paire d'amorces prolongées vers l' arrière contient une région d'amorce universelle appropriée pour différentes plates-formes de séquençage à haut débit.

4. Kit selon la revendication 1, **caractérisé en ce qu'**il y a au moins deux différences de base entre les lieurs.

5. Kit selon la revendication 1, **caractérisé en ce que** les lieurs sont des lieurs de type Y partiellement appariés.

6. Kit selon la revendication 1, **caractérisé en ce que** les gènes pathogènes de surdité présentent des mutations d'insertion, de délétion, de substitution ou de fusion de gènes.

7. Ensemble de paires d'amorces telles que définies dans la revendication 1 pour l'utilisation d'une détection non invasive de mutations de gènes pathogènes de surdité chez un fœtus.

8. Procédé de détection non invasive de mutations de gènes pathogènes de surdité fœtale, comprenant :
-- la conception d'amorces selon une pluralité de locus de mutation prédéterminés de gènes pathogènes de surdité comprenant 22 locus de gène GJB2, de gène GJB3 et de gène SLC26A4 ;
-- le test simultané de la pluralité de locus de mutation prédéterminés de gènes pathogènes de surdité par :
---- la connexion d'ADN plasmatiques contenus dans un échantillon d'ADN plasmatiques obtenu à partir d'une femme enceinte à des lieurs à code à barres de pré-amplification pour obtenir des produits connectés par le marquage des positions de début et terminales des ADN plasmatiques et des 6 bases différentes sur l'extrémité 3' du lieur à code à barres de pré-amplification ;
---- la pré-amplification par PCR du produit connecté pour obtenir des produits pré-amplifiés ;
---- la cyclisation du produit pré-amplifié pour obtenir des ADN cyclisés ;
---- l'amplification par PCR des ADN cyclisés en utilisant les amorces conçues pour obtenir des produits amplifiés ; et
---- le séquençage à haut débit des produits amplifiés et la détection de mutations des gènes pathogènes de surdité fœtale
dans lequel les amorces sont un ensemble de paires d'amorces qui sont prolongées vers l'arrière et sont :
GJB2-F1 (SEQ ID NO: 2): CACGCTGCAGACGATCC,
GJB2-R1 (SEQ ID NO: 3): CCCCAATCCATCTTCTACTCT;
GJB2-F2 (SEQ ID NO: 4): TCCCACATCCGGCTATG,
GJB2-R2 (SEQ ID NO: 5): GATGGGGAAGTAGTGÂTCGTAG;
GJB3-F (SEQ ID NO: 7): CGTGGACTGCTACATTGCC,
GJB3-R (SEQIDNO: 8): ATGTTGGGGCAGGGG;
PDS3-F (SEQ ID NO: 10): CGTCATTTCGGGAGT,
PDS3-R (SEQ ID NO: 11): CTAAGCAGCCATTCC:
PDS5-F (SEQ ID NO: 13): CCCTGACTCTGCTGG,
PDS5-R (SEQ ID NO : 14): CACTGGCAATCAGGA;
PDS7-F2 (SEQ ID NO: 16): TGGCAGTAGCAATTATCGTC,
PDS7-R2 (SEQ ID NO: 17): TTTCATATGGAGCCAACCTG;
PDS10-F (SEQ ID NO : 19): CCACTGCTCTTTCCCGC,
PDS10-R (SEQ ID NO : 20): CAAGAGAAGAATCCTGAGAAGATG ;
PDS17-F (SEQ ID NO : 22): TTCCTGGACGTTGTTGGAG,
PDS17-R (SEQ ID NO : 23): GATATAGCTCCACAGTCAAGCAC; et
PDS19-F (SEQ ID NO : 25): TCTTGAGATTTCACTTGGTT,
PDS19-R (SEQ ID NO : 26): GTTCCATTTTAGAAACGGTA.

9. Procédé selon la revendication 8, dans lequel l'au moins une paire d'amorces prolongées vers l'arrière contient une région d'amorce universelle appropriée pour différentes plates-formes de séquençage à haut débit.

10. Procédé selon la revendication 8, dans lequel il y a au moins deux différences de base entre les lieurs.

11. Procédé selon l'une quelconque des revendications 8 et 9, dans lequel les lieurs sont des lieurs de type Y partiellement appariés.

12. Procédé selon l'une quelconque des revendications 8 à 11, dans lequel la cyclisation des produits pré-amplifiés comprend une cyclisation par attelle, dans lequel des ADN à un seul brin complémentaire aux deux extrémités de l'ADN pré-amplifié sont utilisés comme attelles ; et la fermeture du cycle est effectuée par une ligase Taq thermorésistante.

13. Procédé selon l'une quelconque des revendications 8 à 12, dans lequel la cyclisation des produits pré-amplifiés comprend de multiples réactions d'une unique circulation de système consistant en une dénaturation d'ADN, une hybridation d'ADN d'attelle et une connexion.

14. Procédé selon l'une quelconque des revendications 8 à 13, comprenant en outre la digestion de l'ADN linéaire non cyclisé.

15. Procédé selon l'une quelconque des revendications 8 à 14, dans lequel les mutations de gènes pathogènes de surdité comprennent au moins l'une parmi des mutations d'insertion, de délétion, de substitution ou de fusion de gènes.


### SEQUENCE LISTING

<110> Beijing BerryGenomics Co., Ltd.
<120> A method and a kit for non-invasively detecting fetal deafness pathogenic gene mutations
<160> 31
<170> PatentIn version 3.2
<210> 1
   <211> 417
   <212> DNA
   <213> Homo sapiens
<400> 1
   cgtcttttcc agagcaaacc gcccagagta gaagatggat tggggcacgc tgcagacgat      60
   cctggggggt gtgaacaaac actccaccag cattggaaag atctggctca ccgtcctctt      120
   catttttcgc attatgatcc tcgttgtggc tgcaaaggag gtgtggggag atgagcaggc     180
   cgactttgtc tgcaacaccc tgcagccagg ctgcaagaac gtgtgctacg atcactactt     240
   ccccatctcc cacatccggc tatgggccct gcagctgatc ttcgtgtcca cgccagcgct     300
   cctagtggcc atgcacgtgg cctaccggag acatgagaag aagaggaagt tcatcaaggg     360
   ggagataaag agtgaattta aggacatcga ggagatcaaa acccagaagg tccgcat        417
<210> 2
   <211> 17
   <212> DNA
   <213> Artificial
<400> 2
   cacgctgcag acgatcc 17
<210> 3
   <211> 21
   <212> DNA
   <213> Artificial
<400> 3
   ccccaatcca tcttctactc t 21
<210> 4
   <211> 17
   <212> DNA
   <213> Artificial
<400> 4
   tcccacatcc ggctatg 17
<210> 5
   <211> 22
   <212> DNA
   <213> Artificial
<400> 5
   gatggggaag tagtgatcgt ag 22
<210> 6
   <211> 313
   <212> DNA
   <213> Homo sapiens
<400> 6
   aggcaagaag cacggaggcc tgtggtggac ctacctgttc agcctcatct tcaagctcat      60
   cattgagttc ctcttcctct acctgctgca cactctctgg catggcttca atatgccgcg     120
   cctggtgcag tgtgccaacg tggccccctg ccccaacatc gtggactgct acattgcccg     180
   acctaccgag aagaaaatct tcacctactt catggtgggc gcctccgccg tctgcatcgt     240
   actcaccatc tgtgagctct gctacctcat ctgccacagg gtcctgcgag gcctgcacaa     300
   ggacaagcct cga                                                        313
<210> 7
   <211> 19
   <212> DNA
   <213> Artificial
<400> 7
   cgtggactgc tacattgcc 19
<210> 8
   <211> 15
   <212> DNA
   <213> Artificial
<400> 8
   atgttggggc agggg 15
<210> 9
   <211> 240
   <212> DNA
   <213> Homo sapiens
<400> 9
   tcatgatagt ttagaaaaga tacatctgta gaaaggttga atatttaccg tttctaaaat      60
   ggaaccttga ccctcttgag atttcacttg gttctgtaga tagagtatag catcatggac     120
   cgtcaaaaag aatgtgtcct ttctaatgtt gtcgtcaaag aacccgcatt gctccagctt     180
   ttctatcaca taatcttcaa aagaaagaca caatgttttg ttagttccta ggaaaagaaa     240
<210> 10
   <211> 15
   <212> DNA
   <213> Artificial
<400> 10
   cgtcatttcg ggagt 15
<210> 11
   <211> 15
   <212> DNA
   <213> Artificial
<400> 11
   ctaagcagcc attcc 15
<210> 12
   <211> 300
   <212> DNA
   <213> Homo sapiens
<400> 12
   aatgtataat tcagaaaacc agaaccttac cacccgcagt gatctcactc caacaacgtc      60
   caggaaagat atagctccac agtcaagcac aaggctatgg attggcactt tgggaacgtt     120
   cactttgact ggaagctcag agttccaatc cacttgaatc tctatttcct tggttgggat     180
   atcaagttcc tccagatctt caatatcctc atcaggctca aaagcattat ttgttgaaac     240
   agcatcactt atgatgccat tctaaacgaa gaaaacactg tcaacttaat tgtcaaagat     300
<210> 13
   <211> 15
   <212> DNA
   <213> Artificial
<400> 13
   ccctgactct gctgg 15
<210> 14
   <211> 15
   <212> DNA
   <213> Artificial
<400> 14
   cactggcaat cagga 15
<210> 15
   <211> 336
   <212> DNA
   <213> Homo sapiens
<400> 15
   acgctggttg agatttttca aaatattggt gataccaatc ttgctgattt cactgctgga      60
   ttgctcacca ttgtcgtctg tatggcagtt aaggaattaa atgatcggtt tagacacaaa     120
   atcccagtcc ctattcctat agaagtaatt gtggtaagta gaatatgtag ttagaaagtt     180
   cagcattatt tggttgacaa acaaggaatt attaaaacca atggagtttt taacatcttt     240
   tgttttattt cagacgataa ttgctactgc catttcatat ggagccaacc tggaaaaaaa     300
   ttacaatgct ggcattgtta aatccatccc aagggg                               336
<210> 16
   <211> 20
   <212> DNA
   <213> Artificial
<400> 16
   tggcagtagc aattatcgtc 20
<210> 17
   <211> 20
   <212> DNA
   <213> Artificial
<400> 17
   tttcatatgg agccaacctg 20
<210> 18
   <211> 114
   <212> DNA
   <213> Homo sapiens
<400> 18
   gaattcattg cctttgggat cagcaacatc ttctcaggat tcttctcttg ttttgtggcc 60
   accactgctc tttcccgcac ggccgtccag gagagcactg gaggaaagac acag 114
<210> 19
   <211> 17
   <212> DNA
   <213> Artificial
<400> 19
   ccactgctct ttcccgc 17
<210> 20
   <211> 24
   <212> DNA
   <213> Artificial
<400> 20
   caagagaaga atcctgagaa gatg 24
<210> 21
   <211> 231
   <212> DNA
   <213> Homo sapiens
<400> 21
   aatggcatca taagtgatgc tgtttcaaca aataatgctt ttgagcctga tgaggatatt 60
   gaagatctgg aggaacttga tatcccaacc aaggaaatag agattcaagt ggattggaac 120
   tctgagcttc cagtcaaagt gaacgttccc aaagtgccaa tccatagcct tgtgcttgac 180
   tgtggagcta tatctttcct ggacgttgtt ggagtgagat cactgcgggt g 231
<210> 22
   <211> 19
   <212> DNA
   <213> Artificial
<400> 22
   ttcctggacg ttgttggag 19
<210> 23
   <211> 23
   <212> DNA
   <213> Artificial
<400> 23
   gatatagctc cacagtcaag cac 23
<210> 24
   <211> 218
   <212> DNA
   <213> Homo sapiens
<400> 24
   aaaacattgt gtctttcttt tgaagattat gtgatagaaa agctggagca atgcgggttc 60
   tttgacgaca acattagaaa ggacacattc tttttgacgg tccatgatgc tatactctat 120
   ctacagaacc aagtgaaatc tcaagagggt caaggttcca ttttagaaac ggtaaatatt 180
   caacctttct acagatgtat cttttctaaa ctatcatg 218
<210> 25
   <211> 20
   <212> DNA
   <213> Artificial
<400> 25
   tcttgagatt tcacttggtt 20
<210> 26
   <211> 20
   <212> DNA
   <213> Artificial
<400> 26
   gttccatttt agaaacggta 20
<210> 27
   <211> 16
   <212> DNA
   <213> Artificial
<400> 27
   gtctcatccc tgcgtg 16
<210> 28
   <211> 16
   <212> DNA
   <213> Artificial
<400> 28
   cacgcagggt acgtgt 16
<210> 29
   <211> 16
   <212> DNA
   <213> Artificial
<400> 29
   gtctcatccc tgcgtg 16
<210> 30
   <211> 16
   <212> DNA
   <213> Artificial
<400> 30
   acacgtaccc tgcgtg 16
<210> 31
   <211> 32
   <212> DNA
   <213> Artificial
<400> 31
   gtgcgtccct actctgtgtg catgggacgc ac 32